Europäisches Patentamt

European Patent Office

Office européen des brevets

(19)

(11) Veröffentlichungsnummer: **0 214 976
B1**

(12) **EUROPÄISCHE PATENTSCHRIFT**

(45) Veröffentlichungstag der Patentschrift:
**26.10.88**

(21) Anmeldenummer: **85902460.6**

(22) Anmeldetag: **17.05.85**

(86) Internationale Anmeldenummer:
**PCT/DE 85/00167**

(87) Internationale Veröffentlichungsnummer:
**WO 85/05276 (05.12.85** Gazette 85/26)

(51) Int. Cl.⁴: **A 61 M 16/00**

(54) **VORRICHTUNG ZUM AUFHEIZEN EINES AEROSOLS.**

(30) Priorität: **19.05.84 DE 8415364 U**

(43) Veröffentlichungstag der Anmeldung:
**25.03.87 Patentblatt 87/13**

(45) Bekanntmachung des Hinweises auf die Patenterteilung:
**26.10.88 Patentblatt 88/43**

(84) Benannte Vertragsstaaten:
**AT BE CH DE FR GB IT LI NL SE**

(56) Entgegenhaltungen:
**FR - A - 2 198 758
GB - A - 2 067 415
US - A - 2 524 522
US - A - 4 121 583**

(73) Patentinhaber: **Klarhorst, Günter, Eisgrundstrasse 7,
D-4800 Bielefeld 18 (DE)**

(72) Erfinder: **Klarhorst, Günter, Eisgrundstrasse 7,
D-4800 Bielefeld 18 (DE)**

(74) Vertreter: **Schirmer, Siegfried, Osningstrasse 10,
D-4800 Bielefeld 1 (DE)**

**Beschreibung**

Die Erfindung betrifft eine Vorrichtung zur Erzeugung eines Aerosols, die einen flexiblen spiralförmigen Atemluftschlauch aufweist, über den das von einer Verneblerklammer erzeugte Aerosol zum Patienten führbar ist, wobei zum Aufheizen des erzeugtn Aerosols im Atemluftschlauch mindestens ein Heizdraht angeordnet ist.

Bei den bekannten Vorrichtungen zur Erzeugung eines Aerosols besteht in verschiedenen Anwendungsfällen die Forderung, dass das erzeugte Aerosol in einer bestimmten Temperatur dem Patienten zugeführt wird. Zum Erwärmen des erzeugten Aerosols sind im Hohlraum des Schlauches isolierte Heizdrähte eingelegt. Das Aerosol kommt also direkt mit den Heizdrähten in Berührung. Gemäss DE-A-3 316 322 besteht der Atemluftschlauch aus einem flexiblen Kunststoffschlauch mit glatter Innenwand mit vorzugsweise lose in den Hohlraum eingelegtem Heizwiderstand.

Die Aerosole streichen bei einer solchen Ausführung an den Heizdrähten entlang, wobei an der nicht aufgeheizten Wandung des Atemluftschlauches eine nicht kontrollierbare Abkühlung der Aerosole auftritt. Ausserdem bestehen erhebliche Bedenken in hygienischer Hinsicht. Um eventuelle Infektionen zu vermeiden, müssen die zugeführten Aerosole keimfrei sein. Keimfreie Aerosole lassen sich jedoch nur mit Vorrichtungen erzeugen, die selbst keimfrei arbeiten. Hierzu müssen u.a. der Atemluftschlauch und die lose im Schlauch angeordneten Heizdrähte jeweils in einem Autoklaven oder einem Sterilisator steril gemacht werden. Diese Arbeit ist aufwendig und erfordert grösste Sorgfalt. Eine Gewähr dafür, dass beide Teile stets einwandfrei steril gemacht und in hygienisch einwandfreiem Zustand wieder eingebaut werden, gibt es nicht. Es bleibt überwiegend der Sorgfalt des bedienenden Personals überlassen, dass der Atemluftschlauch und die Heizdrähte völlig steril wieder an das Aerosolgerät angeschlossen werden. Der bekannte Erwärmungseffekt ist inhomogen und kann demzufolge zu Strömungsturbulenzen führen. Wechsende Temperaturen im Atemluftschlauch und Strömungsbehinderungen mit Turbulenzen sind aber wiederum Ursache für Kondensatbildungen, die wegen der Gefahr der Wasseraspiration vermieden werden sollten.

Die Feinstzerstäubung von Medizin im Aerosolgerät bei gleichzeitiger Aufheizung auf eine vorgeschriebene Temperatur ist bei lose in den Atemluftschlauch eingelegten Heizdrähten nicht exakt kontrollierbar. Die exakte Dosierung der Medikamente ist abhängig von der Temperatur und der relativen Feuchte. Eine Abkühlung mit Kondensatbildung im Atemluftschlauch hat ebenso einen Wirkstoffverlust zur Folge wie eine Aufwärmung mit entsprechender Wasseraufsättigung im Bronchialsystem, wenn das Aerosol mit einer niedrigeren Temperatur und einer entsprechenden relativen Fechte bzw. Medikamentenkonzentration zugeführt wird. Die exakte Dosierung der Wirkstoffzufuhr ist somit nur möglich, wenn neben einer garantierten Teilchengrösse der Aerosole auch die Temperatur auf Körpertemperaturniveau mit entsprechender maximaler Feuchte konstant gehalten wird.

Bei einer anderen Ausführung ist zur Aufheizung der Aerosole im Atemluftschlauch zwischen Verneblerklammer und Patientenanschluss ein Heizadapter angeordnet. Um Körpertemperaturniveau beim Eintritt des Aerosols in das Bronchial-System zu gewährleisten und den Abkühlungseffekt auf dem Weg durch den Atemluftschlauch zu kompensieren, muss der Heizadapter das Aerosol zunächst mit einer höheren Temperatur aufheizen. Dadurch wird im Abschnitt der Aufheizung die maximale relative Feuchte unterschnitten und nachfolgend durch Abkühlung Kondensat gebildet, das sich an der Wandung des Atemluftschlauches niederschlägt. Auf diese Weise entsteht auch die Gefahr, dass grössere Wassertropfen mit dem Atemstrom mitgerissen werden.

Aus der FR-A-2 198 758 ist ein Respirator bekannt, mit dem eine hochfeuchte Luft, vorzugsweise nach der Sättigung unter Vermeidung der Bildung von Kondenswasser, geliefert werden soll. Hierzu ist u.a. am Ende des Zuführschlauches ein Temperaturfühler angeordnet, der über einen Temperaturregler die vom beheizbaren Zuführschlauch abgegebene Wärmemenge in vorgegebenen Bereichen regelt. Der Zuführschlauch ist hierbei aus einem flexiblen Rohr aus Kunststoff mit glatter Innen- und glatter Aussenwand gebildet, wobei in der Wandung ein elektrischer Widerstand spiralförmig eingearbeitet ist. Dieser in den Schlauch eingearbeitete Widerstand arbeitet als sekundärer Heizer. Der Gegenstand nach dieser FR-A bezieht sich also auf eine andere Gattung und besitzt keinen spiralförmigen Zuführschlauch.

Die in der US-A-4 121 583 beschriebene Vorrichtung bezieht sich auf ein tragbares Inhaliergerät für Asthma-Kranke. Der Patient inhaliert durch ein Mundstück warme und angefeuchtete Luft, die im Inneren des Generators modifiziert wird. Zwischen jeder Windung eines spiralförmigen Trägers ist eine Heizspirale eingeschlungen, wobei die Heizspirale durch einen zentralen Heizdraht mit einem Abdeckmaterial gebildet ist. Eine Anregung zur Erwärmung eines Aerosols im Atemluftschlauch einer Vorrichtung zur Erzeugung eines Aerosols konnte aus dieser US-A nicht entnommen werden.

Der vorliegenden Erfindung liegt die Aufgabe zugrunde, eine Vorrichtung der aufgezeigten Gattung so auszubilden, dass im gesamten Atemluftschlauch eine gleichmässige Erwärmung des Aerosols gewährleistet ist, ohne dass hierzu das Aerosol direkt mit den zur Erwärmung dienenden Heizdrähten in Berührung kommt, wobei Abweichungen von der vorgewählten Temperatur sofort erkennbar sind.

Diese Aufgabe wird erfindungsgemäss dadurch gelöst, dass der oder die Heizdrähte in den Spiralen des Atemluftschlauches angeordnet sind oder diese bilden und dass zur Anzeige einer Abweichung der am Ausgang des Atemluftschlauches gemessenen Temperatur des abströmenden Aerosols von einer festgelegten Temperatur ein akustischer und/oder ein optischer Signalgeber angeordnet ist. Für die Stromzuführung zu den Heizdrähten kann eine aussenliegende Leitung angeordnet sein, die über einen Stecker am Gehäuse der Vorrichtung anschliessbar ist. Es besteht aber auch die Möglichkeit, die Stromzuführung zu den Heizdrähten an der Aussenseite der Verneblerkammer oder in der Verneblerkammer anzuordnen. Bei dieser Ausführung ist das Stromzuführungskabel zu den Heizdrähten optisch nicht sichtbar. Bei einer Anordnung der Stromzuführung zu den Heizdrähten in der Verneblerkammer bietet es sich an, hierzu die senkrecht verlaufenden Luftkanäle zu benutzen.

Da die Heizdrähte mit einem Schwachstrom von ca. 6-8 Volt gespeist werden, ist zweckmässigerweise im Gehäuse entweder ein entsprechend zugeordneter Transformator oder eine zusätzliche Wicklung an einem bereits vorhandenen Transformator angeordnet.

Durch die Erfindung ist die Möglichkeit geschaffen, das erzeugte Aerosol in einer jeweils festgelegten Temperatur dem Patienten zuzuführen, ohne dass die zur Aufheizung erforderlichen Heizdrähte mit dem Aerosol in Berührung kommen. Im gesamten Atemluftschlauch erfolgt eine gleichmässige Erwärmung des Aerosols. Da Aerosolgeräte auch bei Patienten eingesetzt werden, die selbst nicht in der Lage sind, Veränderungen der Temperatur des zugeführten Aerosols anzuzeigen, ist durch die Anordnung eines akustischen und/oder eines optischen Signalgebers immer die Gewähr gegeben, dass Abweichungen von der vorgegebenen Temperatur sofort erkannt und gegebenenfalls eine Korrektur vorgenommen werden kann.

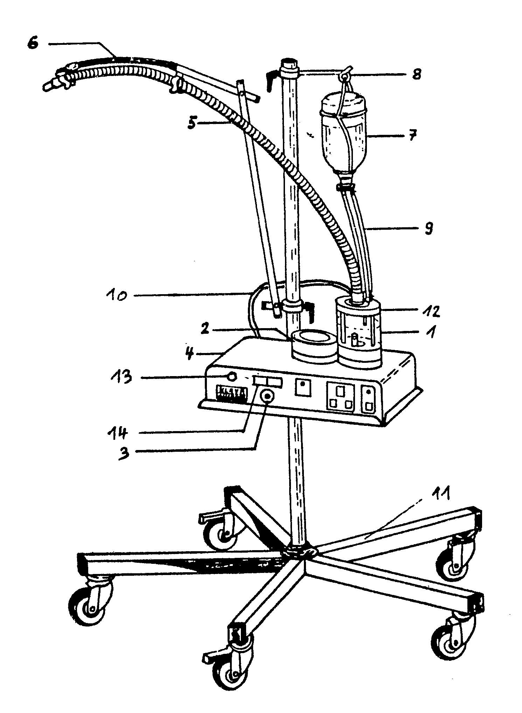

Ein Ausführungsbeispiel der Erfindung ist in der Zeichnung dargestellt und wird im folgenden näher beschrieben. Die einzige Figur zeigt schaubildlich eine Vorrichtung zur Erzeugung eines Aerosols.

Auf einem fahrbaren Unterbau 11 ist das Gehäuse 4 angeordnet. Auf der Oberseite dieses Gehäuses 4 ist eine abnehmbare Verneblerkammer 1 angeordnet. Ein Lüfterrad und ein Bakteriénfilter sind in einer Atemluftkassette 2 zu einer Baueinheit zusammengeschlossen und neben der Verneblerkammer 1 abnehmbar angeordnet. Am Kammerdeckel 12 sind ein von einer in einer Flaschenhalterung 8 befestigten Einweg-Glasinfusionsflasche 7 kommender Verbindungsschlauch 9 und ein zum Patienten führender Atemschlauch 5, der zweckmässigerweise an einem Haltearm 6 geführt ist, angeordnet. Der spiralförmige Atemluftschlauch 5 ist beheizbar ausgebildet. Hierbei dienen die Stahlspiralen des flexiblen Atemluftschlauches 5 als Heizdrähte. Die Stromführung zu den Heizdrähten erfolgt über eine Zuführleitung 10. Über einem am Gehäuse 4 angeordneten Schalter 3 kann die Temperatur eingestellt werden. Der Schalter 13 dient zum Ein- und Ausschalten der Stromzufuhr für die Heizdrähte. Neben einem akustischen und/oder optischen Signalgeber, z.B. in Form einer Blinkleuchte, zur Anzeige einer Abweichung von der festgelegten Temperatur ist auf der Vorderseite des Gehäuses 4 noch eine Temperaturanzeige 14 vorgesehen.

## Patentansprüche

1. Vorrichtung zur Erzeugung eines Aerosols, die einen flexiblen spiralförmigen Atemluftschlauch (5) aufweist, über den das von einer Verneblerkammer (1) erzeugte Aerosol zum Patienten führbar ist, wobei zum Aufheizen des erzeugten Aerosols im Atemluftschlauch (5) mindestens ein Heizdraht angeordnet ist, dadurch gekennzeichnet, dass der oder die Heizdrähte in den Spiralen des Atemluftschlauches (5) angeordnet sind oder diese bilden und dass zur Anzeige einer Abweichung der am Ausgang des Atemluftschlauches (5) gemessenen Temperatur des abströmenden Aerosols von einer festgelegten Temperatur ein akustischer und/oder optischer Signalgeber angeordnet ist.

2. Vorrichtung nach Anspruch 1, dadurch gekennzeichnet, dass für die Stromzuführung zu den Heizdrähten eine aussenliegende Leitung (10) angeordnet ist, die über einen Stecker am Gehäuse (4) der Vorrichtung angeschlossen ist.

3. Vorrichtung nach Anspruch 1, dadurch gekennzeichnet, dass die Stromzuführung zu den Heizdrähten an der Aussenseite der Verneblerkammer (1) oder in der Verneblerkammer (1) angeordnet ist.

4. Vorrichtung nach einem der Ansprüche 1 bis 3, dadurch gekennzeichnet, dass im Gehäuse (4) ein den Heizdrähten zugeordneter Transformator angeordnet ist.

5. Vorrichtung nach einem der Ansprüche 1 bis 3, dadurch gekennzeichnet, dass im Gehäuse (4) eine den Heizdrähten zugeordnete zusätzliche Wicklung an einem Transformator angeordnet ist.

## Claims

1. Device for the production of an aerosol and displaying a flexible spirally shaped breathing air hose (5), by way of which the aerosol produced by an atomiser chamber (1) is conductable to the patient, wherein at least one heating wire for the heating-up of the produced aerosol is arranged in the breathing air hose (5), characterised thereby, that the heating wire or wires is or are arranged in the spirals of the breathing air hose (5) or form these and that an acoustical and/or optical signal transmitter is arranged for the indication of a deviation of the temperature, measured at the exit of the breathing air hose (5), of the outflowing aerosol from a definite temperature.

2. Device according to claim 1, characterised thereby, that an externally disposed line (10), which is connected by way of a plug at the housing (4) of the device, is arranged for the current feed to the heating wires.

3. Device according to claim 1, characterised thereby, that the current feed to the heating wires is arranged at the outward side of the atomiser chamber (1) or in the atomiser chamber (1).

4. Device according to one of the claims 1 to 3, characterised thereby, that a transformer associated with the heating wires is arranged in the housing (4).

5. Device according to one of the claims 1 to 3, characterised thereby, that an additional winding, associated with the heating wires, at a transformator is arranged in the housing (4).

## Revendications

1. Dispositif pour produire un aérosol, pourvu d'un tuyau d'air de respiration (5) flexible et en forme de spirale, par lequel l'aérosol, produit par une chambre de nébulisateur (1), peut être conduit vers le patient, au moins un filament de chauffage étant disposé à cette occasion dans le tuyau d'air de respiration (5), pour chauffer l'aérosol produit, caractérisé en ce que le, ou bien les filaments de chauffage sont disposés dans les spirales du tuyau d'air de respiration (5), ou bien forment celles-ci et qu'un transmetteur de signal, acoustique et/ou optique, est disposé pour indiquer tout écart de la température mesurée de l'aérosol s'écoulant à la sortie du tuyau d'air de respiration (5), par rapport à une température déterminée.

2. Dispositif selon la revendication 1, caractérisé en ce qu'une ligne (10) située à l'extérieur est disposée pour l'amenée de courant aux filaments de chauffage et est raccordée par une prise, sur le carter (4) du dispositif.

3. Dispositif selon la revendication 1, caractérisé en ce que l'amenée de courant aux filaments de chauffage est disposée sur le côté extérieur de la chambre de nébulisateur (1), ou bien dans la chambre de nébulisateur (1).

4. Dispositif selon l'une des revendications 1 à 3, caractérisé en ce qu'un transformateur qui est affecté aux filaments de chauffage est disposé dans le carter (4).

5. Dispositif selon l'une des revendications 1 à 3, caractérisé en ce qu'un enroulement supplémentaire, affecté aux filaments de chauffage, est disposé sur un transformateur, dans le carter (4).